# EUROPEAN PATENT SPECIFICATION

(11) **EP 2 341 932 B1**
(45) Date of publication and mention of the grant of the patent: **09.11.2016**
(21) Application number: 09754195.7
(22) Date of filing: 27.05.2009
(51) Int. Cl.: A61K 39/145, A61K 39/29, A61K 39/39, A61K 39/12, A61N 1/32

(54) **HIGH-ENERGY PULSED ELECTRIC FIELD VACCINE ADJUVANTS**
IMPFSTOFF-ADJUVANTIEN MIT HOCHENERGIE-GEPULSTEM ELEKTROFELD
CHAMP ÉLECTRIQUE PULSÉ DE HAUTE ÉNERGIE FAISANT OFFICE D ADJUVANT VACCINAL

(30) Priority: 27.05.2008 RU 2008121934
(43) Date of publication of application: 13.07.2011
(73) Proprietor: Ooo "Npt Mbp Gormezis", St-petersburg 194064 (RU)
(72) Inventor: ONIKIENKO, Sergey B., St-Petersburg 194356 (RU); ZEMLYANOI, Alexander V., St-Petersburg 192239 (RU); MARGULIS, Boris, A., St-Petersburg 197136 (RU); GUZHOVA, Irina V., St-Petersburg 197136 (RU); PIMENOVA, Anna, A., St-Petersburg 197148 (RU)
(74) Representative: Wells, Andrew
(86) International application number: PCT/IB2009/005744
(87) International publication number: WO 2009/144567

(56) References cited:
- RU-A- 2005 127 615
- US-A1- 2003 170 898
- US-A1- 2006 161 221
- US-A1- 2007 016 125
- STEPHEN J BEEBE ET AL: "Nanosecond pulsed electric fields modulate cell function through intracellular signal transduction mechanisms; Nanosecond pulsed electric fields modulate cell function" PHYSIOLOGICAL MEASUREMENT, INSTITUTE OF PHYSICS PUBLISHING, BRISTOL, GB, vol. 25, no. 4, 1 August 2004 (2004-08-01), pages 1077-1093, XP020074180 ISSN: 0967-3334
- ZHANG XIAOPENG ET AL: "Vaccination with a DNA vaccine based on human PSCA and HSP70 adjuvant enhances the antigen-specific CD8(+) T-cell response and inhibits the PSCA(+) tumors growth in mice" JOURNAL OF GENE MEDICINE, vol. 9, no. 8, August 2007 (2007-08), pages 715-726, XP009116651 ISSN: 1099-498X(print) 1521-2254(ele
- SRIVASTAVA P: "Interaction of heat shock proteins with peptides and antigen presenting cell; chaperoning of the innate and adaptive immune responses" ANNUAL REVIEW OF IMMUNOLOGY, ANNUAL REVIEWS INC, US, vol. 20, 1 January 2002 (2002-01-01), pages 395-425, XP002992707 ISSN: 0732-0582
- EMOHARE OSA ET AL: "Laser-induced thermal stress and the heat shock response in neural cells" ACTA ORTHOPAEDICA SCANDINAVICA, MUNKSGAARD, COPENHAGEN, DK, vol. 75, no. 5, 1 October 2004 (2004-10-01), pages 610-617, XP009116549 ISSN: 0001-6470 cited in the application

## Description

### RELATED APPLICATIONS

This application claims priority to a Russian patent application RU 2008121934, filed on 27 May 2008.

### BACKGROUND OF THE INVENTION

As the use of killed whole or attenuated live vaccines of the previous century gives way to more sophisticated subunit protein and DNA vaccines that will characterize vaccines in next several decades, finding safe and effective vaccine adjuvants becomes increasingly important. Although the new generations of vaccines are more targeted by design, they are also far less immunogenic and typically require powerful adjuvants to induce sufficient immune responses in the human body. Aluminum salts (aluminum hydroxide, aluminum phosphate or alum) have been used in vaccine preparations for over 80 years. While they have the ability to stimulate type II (antibody-mediated) immune responses, they do not induce cytotoxic T-cell or cell-mediated immune responses. They are also incompatible with certain types of vaccine antigens, and are often unable to induce a sufficient immune response to a specific vaccine.

In spite of the need for new vaccine adjuvants, it has been challenging for pharmaceutical companies to identify adjuvants that combine sufficient efficacy and safety in a way that will satisfy the stringent requirements of Western regulatory agencies. Both the FDA and the EMEA have adopted requirements for vaccine adjuvants that are strict and have grown increasingly stringent in recent years. Fundamentally, this orientation is based on the fact that vaccines are used to prevent illness, not treat disease. The safety of a vaccine must be weighed against a hypothetical health threat, in comparison to therapies whose safety profiles are measured against the actual disease they are designed to treat. Very few new chemical or biological adjuvants have approved for human use in the West, and no new type of adjuvant has been approved by the FDA in 80 years.

A wide variety of substances could potentially be used as adjuvants: haptens, hemocyanin, oil-in-water emulsions, surfactants, bacterial and viral components, HLA molecules, cytokines, toll receptor agonists, and heat shock proteins. Many of these are powerful immunostimulators that induce both type I and type II immune responses. However, most also have a high potential for creating side effects and have therefore not been approved for human use. This is, in part, because such adjuvants create a "danger signal" for the body's immune system whose effect often proves difficult to limit. Effective danger signaling using chemical or biological adjuvants almost inevitably results in some degree of reactogenicity or toxicity that is usually unacceptable to regulatory agencies.

An alternate approach to using chemical or biological agents to enhance immune response to vaccination would be to physically manipulate the immune mechanisms of the body at the site of the vaccination, specifically the skin and mucosa, which form the major barrier surfaces to the outside environment and where a significant portion of the body's immune system is located. The physical manipulation of the immune complex in the skin or mucosa would not introduce additional foreign substances into the vaccine site that could trigger more systemic negative responses, and the absence of such foreign substances might have the advantage of limiting the scope of immune enhancement to the extent that harmful reactions would be avoided. While some vaccine delivery systems act as physical adjuvants by causing a degree of damage to the tissues when delivering antigen that leads to local inflammation, these effects are incidental to the function of the devices. There are few devices intentionally designed as physical vaccine adjuvants.

One potential approach to creating a physical adjuvant would be to use electrical fields characterized by extremely short pulses of electrical energy (durations of nanoseconds or shorter) and high energies (intensities of kilovolts per centimeter). Biological cells consist of cytoplasm that is electrically conducting and lipid membranes (both surface membranes and organelle membranes) that act as insulators (dielectrics). When subjected to an electric field, charges accumulate at the cell plasma membrane and the induced potential across the membrane is increased. If the cell is exposed to an electric field of sufficient voltage to exceed a critical value, dielectric breakdown occurs and pores are created in the membrane.

Use of electrical fields to create pores in cell membranes is well known and has been practically applied in the fields of medicine and biology. The size of the pores created in the cell membrane are based on the strength of the electrical field coupled with the nature of the membrane, and the effect is reversible up until a threshold point, at which point the membrane ruptures. Electric fields in the milli- and microsecond range at sufficient intensities (kilovolts per centimeter) are sufficient to create these effects. By modifying the parameters of the electrical field, pores in the cell membrane can be made of sufficient size and duration to allow the passage of a variety of passage of molecules into the cell.

The chief medical application for electroporation is in fact the delivery of drugs and genes to target cells. Electroporation is used to enhance the delivery of chemotherapy drugs into cancer cells, thus increasing the cytotoxic effect of chemotherapy. The first reported clinical use of electroporation-mediated chemotherapy, or electrochemotherapy, was published by Mir *et al.* in 1991 and this therapeutic modality has now been advanced into phase II clinical trials for various cutaneous or subcutaneous cancers, such as melanoma, lymphoma, squamous-cell carcinoma, testicular carcinoma, and malignant pleural effusions. Traditionally, trains of microsecond-duration pulses have been used for electrochemotherapy.

Electroporation to deliver substances into cells in laboratory settings, especially plasmids used for inserting a DNA sequence into a cell, was first described by Wong and Neumann (1982) and Neumann *et al.* (1982), and is now a standard technique in molecular biology and related fields. It is now being used to enhance the delivery of genes to cells, primarily to enhance uptake of DNA vaccines. Electroporation-based DNA vaccination has been demonstrated not only *in vitro* but also in a number of in vivo animal experiments. These studies have shown that electroporation improves the response to DNA vaccination in both the skin and the muscle by enhancing the uptake of the injected plasmids. This technology is now being applied in clinical trials with DNA vaccines in animals, and the initiation of human studies is anticipated.

A different set of biological effects have been discovered from the application of ultrashort (nanosecond and shorter) high-energy pulsed electric fields (usHEPEFs). While electric fields of high field strength and very short pulse duration can also disrupt cell plasma membranes, the targets of usHEPEFs are predominantly the intracellular membrane and structures. When the duration of the electrical pulse applied to the cell is shorter than the charging time of the cell membrane, sufficiently high voltages will induce effects primarily on the intracellular organelle membranes. These intracellular effects depend predominantly on the characteristics of the pulses (number, duration, electric field) and the cell type. At these extremely short pulse durations, electric current is extremely small and therefore thermal effects from energy transfer to cells or tissues is negligible.

These usHEPEFs can alter the function of cells first through poration of cell organelles such as the endoplasmic reticulum or mitochondria in a fashion similar to that of poration of cell membranes. This phenomenon is sometimes defined as "nanoelectroporation." Compared to microsecond pulses that porate cell membranes, the effects of nanosecond pulses on organelle membranes are relatively short-lived - a matter of seconds or less rather than minutes. At these durations, nanoelectroporation can elicit effects normally associated with internal cell biological signaling. Current medical applications of nanoelectroporation are directed mainly toward treating cancer, since nanoelectroporation can induce signaling cascades inside cells that more readily lead to apoptosis in abnormal cells (U.S. Patent 6,326,177 B1; U.S. Patent Application 2002/0010491 A1). Most studies of nanoelectroporation have been performed *in vitro,* but a few animal studies have been recently published and Garon's group reported results in one human patient with melanoma. The advance of research in this area has been slowed in part by the complexity of the devices required to produce these effects. No commercially-available devices are yet available.

Further investigations have demonstrated that usHEPEFs at very short pulse durations (< 100 nanoseconds) and at electric field strengths below the threshold for inducing nanoelectroporation can also induce intracellular effects. This is especially the case for pulses in the range ≤10 nanoseconds. Intercellular effects generated without significant poration of intracellular membranes is sometimes referred to as "electroperturbation" (U.S. Patent Application 2003/0170898). The exploration of biological and medical applications of electroperturbation is very much in the exploration phase. Some of the effects described in the literature and noted throughout this document include calcium fluxes, release of cytochrome c, triggering of caspase- based pathways, alteration of DNA, and relocation of phosphatidylserine. Variation of the pulse duration and electric field strength exerted on cells may have opposite effects, depending on the parameters. Many of these effects have been described in studies of cancer cells and result in the destruction of the cell itself. In a few cases positive effects on cell function have been described that have application to medical therapy, such as the activation of platelet aggregation through induction of calcium flux (US Patent Application 2006/0161221).

Beebe S J at al, Physiological Measurement, Institute of Physics Publishing, Bristol GB, vol 25, no.4, 1 August 2004, pp1077-1093, describes the effects of nanosecond pulsed electric fields on mammalian cell structure and function.

A currently unexplored area of research on usHEPEFs, particularly in the area of electroperturbation, is the effect of these electrical fields on the function of the immune system itself, particularly the immune complex in the skin or mucosa. While studies have shown that electroporation can improve DNA vaccines by increasing the uptake of DNA into cells, this is a vaccine delivery strategy whose utility has empirically been limited to transferance of genetic material. The ability of usHEPEFs to alter the function of the immune system itself has not been previously described in the literature, nor has this phenomena been harnessed for practical purposes.

### SUMMARY OF THE INVENTION

The present invention is directed to the enhancement of vaccination through the application of usHEPEFs capable of enhancing the immune response to vaccination. The invention is related to the use of devices capable of generating usHEPEFs that enhance the immune response to vaccination, referred to here as a pulsed electric field vaccine adjuvant (PEVA). A PEVA device administers usHEPEFs to the skin or mucosal layers of a subject, where the electrical pulses can improve the response of the local immune complex to vaccination. This approach is distinct from the target of current vaccine electroporation devices, which largely target the myocytes of the muscles. When specific parameters of usHEPEFs are applied to the skin or mucosal layers of a subject using a PEVA device, it can improve the response of the subject to vaccination.

Accordingly, the invention provides use of an antigen in the manufacture of a vaccine, wherein the vaccine is for administration in combination with a pulsed electrical field of sufficient voltage and pulse frequency for enhancing an immunostimulatory effect of the vaccine as compared to the vaccine used without a pulsed electrical field, but below a threshold of electroporation of cell membranes, and wherein the administration comprises exposing an area of skin or mucosa of a subject to ultrashort, high energy, pulsed electrical fields, and contacting the subject with the antigen in the layers of the skin or mucosa at or near the site of the electrical field.

In one aspect, the electrical field does not cause apoptosis or necrosis to more than 1% of the cells. In one aspect, the duration of the electrical field pulses is under 100 nanoseconds. In one aspect, the electrical field ranges from about 1kV/cm to about 300 kV/cm.

The invention also provides an antigen for use in a vaccine, wherein the vaccine is for administration in combination with a pulsed electrical field of sufficient voltage and pulse frequency for enhancing an immunostimulatory effect of the vaccine as compared to the vaccine used without a pulsed electrical field, but below a threshold of electroporation of cell membranes, and wherein the administration comprises exposing an area of skin or mucosa of a subject to ultrashort, high energy, pulsed electrical fields, and contacting the subject with the antigen in the layers of the skin or mucosa at or near the site of the electrical field.

In one aspect, the duration of the electrical field pulses is under 100 nanoseconds. In one aspect, the electrical field ranges from about 1kV/cm to about 300 kV/cm.

The present disclosure describes methods of administering a vaccine to a subject including exposing an area of skin or mucosa of the subject a series of ultrashort, high energy, pulsed electrical fields wherein the electrical fields do not cause visible damage to the skin or mucosal surface subject to the electric field or significant or irreversible damage to cells exposed to the discharge, and contacting the subject with the antigen in the layers of the skin or mucosa at or near the site of the electrical field, thereby administering the vaccine. Irreversible damage to the cells includes cell death, either upon exposure to the electrical field, or as a direct result of the electrical field. Significant damage is damage to at least 1%, 0.5%, 0.25%, 0.1%, 0.01%, or 0.001% of cells exposed to the electrical field.

The disclosure further describes methods to increase the response of the subject to the vaccine by increasing the release of heat shock protein 70 from cells in the area of exposure to the electrical field, activating or maturing the immune cells in the area of exposure to the electrical field, and/or concentrating immune cells at or near the site of exposure to the electric field compared to a site not exposed to and electric field by exposing an epidermal layer of the skin or mucosa to an electric field wherein the electric field does not cause visible damage to the skin or mucosal surface or cause significant or irreversible damage to cells in the exposed area.

In the methods of the disclosure, the duration of the electric field pulses are below 100 nanoseconds, about 1 to 100 nanoseconds, about 1 to 50 nanoseconds, about 30 nanoseconds, or in the 100 picosecond range. These individual electric field pulses are below the charging time of the cell membrane, which is typically 75-100 nanoseconds. The combination of electric field pulse, voltage, and frequency is below the threshold required for electroporation of the cell organelle membranes, and exposure of the tissue to the electrical field ranges from a matter of seconds to several minutes. In a setup typically used to induce nanoelectroporation, such long trains of pulses (typically in the range of 10,000 or more) would lead to irreversible intracellular membrane poration, leading to significant cell damage and death. The invention is therefore distinguished from other examples of the art by its nondestructive nature to the vast majority of cells within an exposed tissue.

In the methods of the disclosure, the electric field is administered as close as possible to the time of the vaccine, either before or after administration of the vaccine, to within about an hour, within about 45 minutes, within about 30 minutes, within about 15 minutes, to within about 10 minutes of vaccine adminstration.

The methods of the disclosure include administering a vaccine to an epidermal, dermal or subcutaneous layer of skin or to the epithelial mucosa of the subject to provide an increase in a detectable response to the vaccine as compared to administering a vaccine to an area of the epidermal, dermal or subcutaneous layer of skin or to the epithelial mucosal layer of a subject not exposed to an electrical field. In the methods of the disclosure, an increase in a detectable response can include an increase in antibody titer specific to the vaccine, an increase in resistance to a condition as demonstrated, for example by a decrease in infection rate upon exposure to a pathogen specific to the vaccine, a decrease in mortality in response to exposure to a pathogen specific to the vaccine, and/or a decrease in time to detect a response to the vaccine.

The methods of the disclosure can further include administration of the vaccine to a surface of the body that has a similar complement of immune cells compared to the skin, including but not limited to mucosal surfaces such as the oral cavity, the nasal cavity, the lining of the intestines, or the lining of the vaginal canal.

The methods of the disclosure can further include detecting a response to the vaccine. In other instances, the disclosure can also include identifying a subject in need of vaccination. In some instances of the disclosure, the subject is immunocompromised. In some instances, the disclosure further includes co-administration of the vaccine with heat shock protein 70.

The disclosure describes methods that can be practiced using an electric field device that provides the necessary parameters of energy and power that can stimulate an immune response to an antigen as a result of the stimulation of immune cells in the
skin or mucosa. The immune- enhancing effect of these electrical fields can be distinguished from electroporation as currently practiced because it does not require electric field pulse durations as long as those typically required for electroporation (typically greater than 100 nanoseconds), does not require the
insertion of needles into the skin or mucosa to deliver an electroporating dose, is not delivered to the muscle of subjects exposed to an electrical field, and can be used with all types of vaccines, including peptide, protein, attenuated live vaccines, as well as DNA vaccines. Furthermore, the immune-enhancing effects of these electric fields can be distinguished from nanoelectroporation as currently practices because it does not require a combination of electric field strength and pulse duration sufficient to open pores in cellular organelles and can be applied to a tissue for durations far longer than can be used with nanoelectroporation without causing significant tissue damage.

The disclosure describes methods for contacting a subject with a vaccine by injection or other types of delivery at or near the site of exposure to the electrical field. The disclosure describes methods for injection including, but not limited to intradermal, subcutaneous, and intramuscular injection. In addition, the disclosure provides for the use of other forms of vaccine delivery, including diffusion of the vaccine antigen from the skin or mucosal surface in the form of a liquid or aerosol.

### BRIEF DESCRIPTION OF THE DRAWINGS

Fig. 1 shows a teratron for delivery of high voltage, nanosecond electrical pulses to the gas lasing unit of a copper-vapor laser system.
Fig. 2 shows a semiconductor-based electric field generator produced by the Russian Federal Nuclear Center - The All-Russian Research Institute of Experimental Physics (RFNC - VNIIEF), Sarov, Nizhny Novgorod region, Russia.
Fig. 3 shows a small-sized generator capable of producing high energy, nanosecond pulses based on the SOS diodes produced by the Institute of Electrophysics of the Ural Division of the Russian Academy of Sciences (IEP UD RAS), Ekaterinburg, Russia.
Fig. 4 shows a coaxially-situated electrode with insulators used to conduct ultrashort, high-energy electric pulses to the skin of subjects, average air gap approximately 5 mm.
Fig. 5 shows a set of concentrically-arranged electrodes encased in a glass insulator. This electrode is typically used with Darsenval physiotherapy treatment devices.
Fig. 6 shows an electrode device based on the array of the cone-shaped electrodes.
Fig. 7 shows a graph showing the inverse relationship between electric field strength and pulse duration required for nanoelectroporation of cellular organelles.
Fig. 8 shows the results of immunohistochemical staining for heat shock protein 70 outside of epidermal cells from the ventral sheets of mouse ear tissue. The quantity of HSP found in the extracellular space was approximately three times that of ventral sheet tissue from the control (non-exposed) mouse ear tissue.

### DEFINITIONS

As used herein "treating" a disease in a subject or "treating" a subject having a disease refers to subjecting the subject to a pharmaceutical treatment, e.g., the administration of a drug, such that the extent of the disease is decreased or prevented. For example, treating results in the reduction of at least on sign or symptom of the disease or condition. Treatment includes (but is not limited to) administration of a composition, such as a pharmaceutical composition, in combination with exposure to an electric field as described herein, and may be performed either prophylactically or subsequent to the initiation of a pathologic event. Treatment can require administration of an agent and/ or treatment more than once.

As used herein, "preventing" as it refers to a condition means that a subject in which the occurrence of a condition is prevented, will show no detectable symptoms of a condition of interest. No detectable symptoms means less than 10%, 5%, 1.0%, 0.5%, 0.1% or 0.01% of the level of detectable symptoms of a subject that has been diagnosed with a condition of interest.

As used herein, a "condition" includes any abnormality that can occur in a subject including any disease, infection, disorder, tumor, cancer, inflammatory condition, or cellular structure associated with disordered function. To "prevent the occurrence of a condition" also means to stop or delay the occurrence of a condition of interest. Prevention can require administration of an agent and/ or treatment more than once.

As used herein, "significant and irreversible damage" is understood as causing harm to the cells which directly leads to death of the cells exposed to the electric field in the methods of the invention. Irreversible damage to a cell means that the function of the cell is negatively altered or compromised and cannot return to normal. Irreversible damage can occur when the cell is destroyed, physically removed from its environment, or when the function of the cell is compromised, for example by irreversible disruption of the membrane or destruction or damage to one or more of the organelles or processes within the cell. Significant damage refers to the number of cells that are killed, i.e., irreversibly damaged, either as a ratio (percent) of the cells exposed to the electrical field, as a defined number of cells per electric field exposure, or a defined area of cells. Significant damage is damage to at least 1%. 0.5%, 0.25%. 0.1%, 0.001%, or 0.0001% of the cells expose to the electrical field.

As used herein, "adjuvant" is understood as an aid or contributor to increase the efficacy or potency of a vaccine or in the prevention, amelioration, or cure of disease by increasing the efficacy or potency of a therapeutic agent as compared to a vaccine or agent administered without the adjuvant. An increase in the efficacy or potency can include a decrease in the amount of vaccine or agent to be administered, a decrease in the frequency and/or number of doses to be administered, or a more rapid or robust response to the agent or vaccine (i.e., higher antibody titer). An adjuvant can be an agent or a device.

As used herein, "subject" refers to a mammal. A human subject can be known as a patient.

As used herein, "mammal" refers to any mammal including but not limited to human, mouse, rat, sheep, monkey, goat, rabbit, hamster, horse, cow or pig.

A "non-human mammal", as used herein, refers to any mammal that is not a human.

As used herein "exposing" means treating with an electric field for an exposure time useful to the invention. In one instance, exposing means to expose the skin or mucosa of a subject to an electric field applied in a pulse or a series of pulses, wherein the pulse is applied for a particular duration. The range of pulse durations are in the hundreds of picoseconds to 100 nanoseconds (for example, about 100, 200, 300, 400, 500, 600, 700, 800, 900 picoseconds, or about 1, 2, 3, 4, 5, 6, 7, 8, 9, 10, 12, 15, 20, 25, 30, 35, 40, 45, 50, 75, or 100 nanoseconds). It is understood that the actual pulse length will vary somewhat based on the limitations of the electrical device and its ability to generate consistent pulse lengths.

In another instance, "exposing" means to expose a subject to an electric field of a particular field strength. Optimal voltage intensities produced by the device range from about 1 kV/cm to 300 kV/cm (for example, 1, 2, 3, 4, 5, 10, 20, 30, 40, 50, 100, 200 or 300kV/cm). It is understood that the actual electric field strength will vary somewhat based on the limitations of the electrical device. In another instance, "exposing" means to expose a subject to an electric field with a particular pattern of electric field discharges. The types of patterns of exposure can include a single pulse, a series of single pulses separated by a matter of seconds, or a train of pulses lasting for a number of seconds. Typical exposure patterns are in the range of 30-90 seconds. In another instance, "expose" means to expose a particular area of the subject. Typical exposure areas are in the range of a less than a square centimeter of skin or mucosa.

In one instance, the exposure to an electric field occurs prior to administration of said agent. The exposure to said electric field occurs about 30 seconds, about 5 minutes or about one or more hours before administration of said agent.

In another instance, the exposure to an electric field occurs after the administration of said agent. The exposure to said electric field occurs about 30 seconds, about 5 minutes or about 1 or more hours after administration of said agent.

As used herein, an electric field refers to a field of force around a charged particle or the creation of a voltage potential across a membrane.

An electric field pulse refers to the creation of an electric field for a distinct period of time, in this case for periods of nanoseconds or shorter. The electric field strength is a measurement of the voltage potential across two electrodes, typically measured in volts per meter or kilovolts per centimeter.

As used herein, high-energy refers to the electric field strength between two electrodes of a kilovolt per centimeter or greater.

As used herein, ultrashort refers to durations below the microsecond level, including but not limited to the nanosecond and picoseconds range.

As used herein, electroporation refers to the use of an electric field to cause the rearrangement of the structure of the membrane or membranes of cells and/or their organelles in a way that introduces or modifies the porosity of the membrane(s), creating a means by which fluid or other substances may be transported across the membrane into or out of the cell or organelle that the membrane surrounds. The use of ultrashort electric field pulses to induce such changes is referred to as "nanoelectroporation" and typically effect cell organelle membranes.

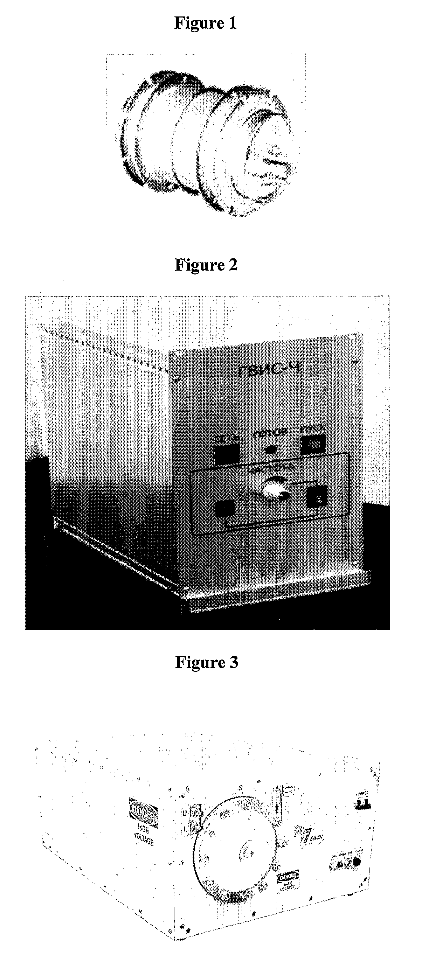

As used herein, a pulsed electric field vaccine adjuvant (PEVA) device includes any electrical device that is currently available or may become available that can provide the appropriate voltage, pulse duration, frequency, and power required by the methods of the instant disclosure. In one instance , a laser teratron capable of generating nanosecond pulsed, high energy electric fields is used (Figure 1). In another instance, a semiconductor-based electric field generator capable of generating high-frequency electric fields between 1-40 kV/cm, pulses in the range of 6 and 30 nanoseconds, frequencies between 1 and 500 Hertz, and currents below 100 microamps is used (Figure 2, Spirov et al. Russian Patent Application A2007113543). In another instance a small-sized generator capable of producing high energy, nanosecond pulses based on the SOS diodes is used with electric fields between 100-250 kV/cm, pulse durations from 3-5 nanoseconds, and frequencies of 300 Hertz (Figure 3).

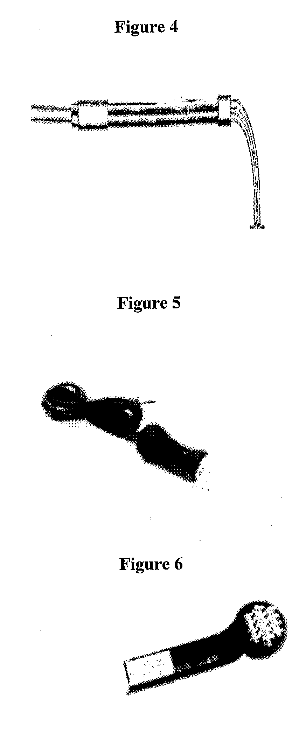

As used herein, an electrode refers to a terminal through which an electric current passes between metallic and nonmetallic parts of an electric circuit. Typically, electrodes are used in pairs to create an electric field potential across the gap between the electrodes. Cells or tissues are exposed to the electric field generated between the electrodes. In one instance, electrodes used with the PEVA device are coaxially-situated probes separated by insulators with high dielectric permeability in the range of five times that of air (Figure 4). In another instance, the electrodes form two concentric rings separated by an air gap. The electrodes are enclosed by a glass insulator and do not come in contact with the skin. These electrodes are typically used in Darsenval physiotherapy treatment devices (Figure 5). In yet another instance, the electrode device (Figure 6) is based on the array of the cone-shaped electrodes.

As used herein, "administering" refers to any method according to the disclosure including but not limited to injection, subcutaneous, transcutaneous, intramuscular, mucosal, intraperitoneal, intracranial and spinal injection, administration directly to a blood vessel, including artery, vein or capillary, intravenous drip, ingestion via the oral route, inhalation, transepithelial diffusion (such as via a drug-impregnated, adhesive patch) or by the use of an implantable, time-release drug delivery device, which may comprise a reservoir of exogenously-produced agent or may, instead, comprise cells that produce and secrete the therapeutic agent or topical application. Additional methods of administration are provided herein below in the section entitled "Dosage and Administration." As used herein, "administering" can also refer to exposure of a subject to an electrical field, preferably for a therapeutic use.

As used herein, "contacting" means exposing a subject to, for example by any of the methods of administration described herein. "Contacting" refers to exposing a subject to, for example, an agent for a duration of about 1, 5, 10, 20, 30, 40, 50 minutes, about 1, 2, 5, 10, 20,
24 hours, about 2, 3, 4, 5, 6, 7, 8, 9, 10 days or more. In one instance, "contacting" refers to exposing a subject more than once, for example about 2, 3, 4, 5, 6, 7, 8, 9, 10 times or more.

As used herein, an "agent" refers to any protein, recombinant protein, small molecule, DNA, RNA, antigen, parasite, virus, bacteria, or other prokaryotic or eukaryotic cells, either whole cells or fragments thereof, or combination thereof.

An "agent" also includes a vaccine. A used herein, a "vaccine" is a preparation which is used to increase immunity of a subject to a particular condition or antigen.

An "antigen" is understood as any compound that can be used to stimulate a specific immune response. An antigen can be an isolated or purified protein, nucleic acid, carbohydrate, small molecule, and the like. Alternatively, an antigen can be a complex mixture, naturally or artificially generated including a mixture of one or more of protein, nucleic acid, carbohydrate, small molecule optionally in the form of a pathogen, particularly a killed or attenuated pathogen. Antigens include self- and non-self antigens. For example, an antigen can be a protein that is not normally present in a subject, e.g., a cancer cell. An antigen can also be a contraceptive protein (e.g., riboflavin carrier protein).

A "nucleic acid therapeutic" or "nucleic acid antagonist" can be any nucleic acid (DNA, RNA, or a combination thereof) or an analog thereof (e.g., PNA) optionally including one or more modifications (see, e.g., US Patents 7,015,315 and 6,670,461,) to modulate pharmacokinetic or pharmacodynamic properties of the nucleic acid. Nucleic acid antagonists can be antisense oligonucleotides (see, e.g., US Patents 5,366,878; or 6,921,812,), small interfering (si)RNA (see, e.g., US Patent 7,056,704,), short hairpin RNA (see, e.g., US Patent publication 20080119427,), or other double stranded RNA molecules (see, e.g., US Patent publication 20070265220,). Nucleic acid antagonists are well known in the art.

A "small molecule" is understood as is meant a compound having a molecular weight of no more than about 1500 daltons, 1000 daltons, 750 daltons, 500 daltons. A small molecule is not a nucleic acid or polypeptide.

By "analog" is meant a molecule that is not identical, but has analogous functional or structural features.

As used herein, "increase" as it refers to a response means elevating the level of response (for example, cell membrane permeability, production or secretion of heat shock proteins (HSPs), increased concentration, maturation and/or activation of dendritic cells at the site of exposure to the electrical field) following administration of an agent or antigen by at least about 2-fold (for example about 2, 3, 4, 5, 10, 15, 20, 25, 30, 35, 40, 45, 50, 55, 60, 65, 70, 75, 80, 85, 90, 95, 100, 1000-fold or more) or at least about 2% (for example, about 2, 3, 4, 5, 10, 5, 20, 25, 30, 35, 40, 45, 50, 55, 60, 65, 70, 75, 80, 85, 90, 95, 99, or 100%), in a subject that has been exposed to an electrical field, as compared to an untreated subject.

As used herein, a "heat shock protein" refers to a group of proteins whose expression is increased when cells are exposed to elevated temperatures or other stress. The HSPs are named according to their molecular weights. For example, HSP 60, HSP 70 and HSP 90 (the most widely-studied HSPs) refer to families of heat shock proteins on the order of 60, 70 and 90 kilodaltons in size, respectively. The small 8 kilodalton protein ubiquitin, which marks proteins for degradation, also has features of a heat shock protein.

As used herein, a "detectable response" includes a discernable, preferably a measurable level of a response that occurs in a subject that has been exposed to an electrical field, as described herein, but not in a subject that has not been exposed to an electrical field. A "response" that is detected includes, but is not limited to, one or more of an increase in immunogenicity, an increase in an immune response, an increase in the concentration of dendritic cells at the site or irradiation, an increase in the maturation or activation of dendritic cells, an increase in the number of activated or mature dendritic cells, an increase in the production of HSPs, an increase in the secretion of HSPs, an increase in the level of one or more cytokines or chemokines, an increase in antibody titer to an agent, or an increase in lymphocyte cytotoxic activity, according to the methods described herein, an increase in response to antigen challenge, or an increase in viability in response to pathogen challenge.

A "detectable response" means a response that is at least about 0.01%, 0.5%, 1%, 10%, 20%, 30% or more than the response of a subject that has not been exposed to an electrical field. A "detectable response" also means a response that is at least about 0.5, 1, 2, 3, 4, 5, 10, 20, 30, 40, 50, 60, 70, 80, 90, 100, 1000-fold or more greater than the response of a subject that has not been exposed to an electrical field.

As used herein, "detecting a response" means performing an assay to determine if a response has occurred. In an embodiment, the response can be zero or below the limit of letection of the assay method.

As used herein, "immunogenicity" refers to the ability, for example the ability of an agent, to induce humoral and/or cell-mediated immune responses in a subject.

As used herein, "immune response" refers to a response made by the immune system of an organism to a substance, which includes but is not limited to foreign or self proteins. There are three general types of "immune response" including, but not limited to mucosal, humoral, and cellular "immune responses."

A "mucosal immune response" results from the production of secretory IgA (sIgA) antibodies in secretions that bathe all mucosal surfaces of the respiratory tract, gastrointestinal tract and the genitourinary tract and in secretions from all secretory glands. These sIgA antibodies act to prevent colonization of pathogens on a mucosal surface and thus act as a first ine of defense to prevent colonization or invasion through a mucosal surface. The production of sIgA can be stimulated either by local immunization of the secretory gland or tissue or by presentation of an antigen to either the gut-associated lymphoid tissue (GALT or Peyer's patches) )r the bronchial-associated lymphoid tissue (BALT). Membranous microfold cells, otherwise known as M cells, cover the surface of the GALT and BALT and may be associated with other secretory mucosal surfaces. M cells act to sample antigens from the luminal space adjacent to the mucosal surface and transfer such antigens to antigen-presenting cells (dendritic cells and macrophages), which in turn present the antigen to a T lymphocyte (in the case of T-dependent antigens), which process the antigen for presentation to a committed B cell. B cells are then stimulated to proliferate, migrate and ultimately be transformed into an antibody-secreting plasma cell producing IgA against the presented antigen.
When the antigen is taken up by M cells overlying the GALT and BALT, a generalized mucosal immunity results with sIgA against the antigen being produced by all secretory tissues in the body. Oral immunization is therefore an important route to stimulate a generalized mucosal immune response and, in addition, leads to local stimulation of a secretory immune response in the oral cavity and in the gastrointestinal tract.

An "immune response" can be measured using a technique known to those of skill in the art. For example, serum, blood or other secretions may be obtained from an organism for which an "immune response" is suspected to be present, and assayed for the presence of a of the above mentioned immunoglobulins using an enzyme-linked immuno-absorbant assay (ELISA; U.S. Pat. No. 5,951,988). According to the present disclosure, an antigen can be said to stimulate an "immune response" if the quantitative measure of immunoglobulins in an animal treated with an antigen detected by ELISA is statistically different from the measure of immunoglobulins detected in an animal not treated with the antigen, wherein the immunoglobulins are specific for the antigen. A statistical test known in the art may be used to determine the difference in measured immunoglobulin levels including, but not limited to ANOVA, Student's T-test, and the like, wherein the P value is at least <0.1, <0.05, <0.01, 0.005, 0.001, and even 0.0001.

An "immune response" can be measured using other techniques such as immunohistochemistry using labeled antibodies which are specific for portions of the immunoglobulins raised during the "immune response". Tissue from an animal to which an antigen has been administered according to the disclosure may be obtained and processed for immunohistochemistry using techniques well known in the art. Microscopic data obtained by immunohistochemistry can be quantitated by scanning the immunohistochemically stained tissue sample and quantitating the level of staining using a computer software program known to those of skill in the art including, but not limited to NIH Image (National Institutes of Health, Bethesda, MD). According to the present disclosure, an antigen of the present disclosure can be said to stimulate an "immune response" if the quantitative measure of immunohistochemical staining in an animal treated with an antigen is statistically different from the measure of immunohistochemical staining detected in an animal not treated with the same antigen, wherein said histochemical staining requires binding specific for that protein. A statistical test known in the art may be used to determine the difference in measured immunohistochemical staining levels including, but not limited to ANOVA, Student's T-test, and the like, wherein the P value is at least <0.1, <0.05, <0.01, 0.005, 0.001, and even 0.0001.

As used herein, "dendritic cells" refers to antigen-processing and presenting cells that are involved in a variety of T cell responses including priming of naïve T cells. In an immature state, dendritic cells are efficient at taking up extracellular antigen. In a mature state, they are highly efficient at presenting these antigens to T cells. The two major types of dendritic cells in the skin are Langerhans cells and dermal dendritic cells. Langerhans cells are have dark nuclei and pale or clear cytoplasm, no desmosomes attaching to neighboring cells, no tonofilament bundles and no melanosomes. However, they do contain smooth vesicles, multivesicular bodies, and lysosomes, but most characteristic are the Birbeck granules. They are also characterized by the expression of the type II transmembrane Ca²⁺-dependent lectin, langerin/CD207. Immature Langerhans cells are present in the basal, spinous and granular layers of the skin, but are also found in other squamous epithelia, including the oral cavity, esophagus, and vagina, as well as in lymphoid organs such as the spleen, thymus, and lymph node. Langerhans cells were traditionally thought to develop from circulating haematopoietic precursor cells originating from the bone marrow, but recent studies suggest they are normally derived from a local pool of proliferating host haematopoietic precursor cells in the skin. Once the precursor cells differentiate into Langerhans cells, they remain relatively fixed in place until they undergo activation and maturation in response to antigen uptake or chemical signals.

Dermal dendritic cells are a more diverse group of antigen-presenting cells that do not possess the distinctive characteristics or cell markers of Langerhans cells and originate from precursors circulating in the blood. They tend to be found in the dermal layer of the skin and are much more mobile relative to Langerhans cells. Both types of antigen-presenting cells can migrate to nearby lymph nodes after activation and maturation, but do not migrate to the same parts of the lymph nodes. During inflammation of the skin, monocytes may travel to the skin from circulation, performing similar roles to the resident dendritic cells. In addition to the skin, antigen-presenting dendritic cells are found in other mucosal sites such as the respiratory tract and the gastrointestinal tract, where they play similar roles.

As used herein an "activated dendritic cell" refers to dendritic cells that have phagocytosed antigens, have increased their expression of major histocompatibility complex class I or II and costimulatory molecules, and will migrate to T cell areas of draining lymph nodes. Activated dendritic cells undergo a maturation process that allows them to become stimulators of T cell immunity. As used herein a "mature dendritic cell" refers to cells that have gained potent immunogenic capacity, including the ability to secrete T-cell attracting chemokines and to interact with T-cells. They are often characterized as having the properties of being CD24+, CD25+, CD69-, CD80 (B7-1)+, CD83+, CD122+ and CCR7+. It is understood that not all cells necessarily express, or do not express, detectable levels of all of the markers listed. Mature dendritic cells lose the capacity to phagocytose antigen. Differences in types of dendritic cells can usually be detected with a number of immunohistochemistry assays.

As used herein, skin refers to the body's outer covering. It is made up of two main layers; the epidermis and the dermis. The epidermis contains five layers. From top to bottom the layers are called the stratum corneum, the stratum licidum, the stratum granulosum, the stratum spinosum and the stratum basale. At the stratum basale, cells are shaped like columns. These cells divide and push already formed cells into higher layers. As the cells move into the higher layers, they flatten and eventually die. The top layer of the epidermis, the stratum corneum, is made of dead, flat skin cells that shed about every 2 weeks. The epidermis contains specialized cells including the melanocytes, which produce and contain pigment (melanin), the Langerhans cells, and the Merkel's cells. The thickness of the epidermis varies in different types of skin. It is the thinnest on the eyelids at .05 mm and the thickest on the palms and soles at 1.5 mm.

The dermis contains two layers: the upper papillary and lower reticular layers. The upper papillary layer contains a thin arrangement of collagen fibers. The lower, reticular layer, is thicker and made of thick collagen fibers that are arranged parallel to the surface of the skin. The dermis contains three main kinds of tissues-collagen, elastic tissue, and reticular fibers, all of which are distributed throughout the dermis, as well as several specialized structures: the blood and lymph vessels, hair follicles, and glands. These glands produce sweat, which helps regulate body temperature, and sebum, an oily substance that helps keep the skin from drying out. Sweat and sebum reach the skin's surface through tiny openings called pores. There are also specialized nerve cells called Meissner's and Vater-Pacini corpuscles that transmit the sensations of touch and pressure. The dermis also varies in thickness depending on the location of the skin. It is .3 mm on the eyelid and 3.0 mm on the back. The dermis is composed of three types of tissue that are present throughout - not in layers.

As used herein, the mucosa refers to the moist layer that lines the internal passages of the body exposed to the outside environment: the mouth, nose, throat, esophagus and lungs, the inside of the eyelids, the gastrointestinal tract and the vagina. In general the mucosa is composed of two types of layers, the outer epithelial layer and inner lamina propria. Different mucosal tissues contain specialized tissues. For example, the epithelial layer of the trachea contain ciliar cells that are designed to propel mucosa up and out of the lung area. In the digestive tract, the mucosa also includes a layer of smooth muscle called the muscularis mucosae. Specific mucosal tissues also contain the specialized immune complexes described earlier (BALT and GALT).

As used herein, "identifying" as it refers detecting indicators that a subject that is susceptible to a condition refers to the process of assessing a subject and determining that the subject is at risk of developing or could develop a condition. A subject can be susceptible to a condition due to environmental conditions (e.g., population density, availability of clean water, hospitalization), genetic predisposition (e.g., heritable immune deficiency), the presence of other condition (e.g., burns or other injury in which the skin is disrupted, cystic fibrosis, AIDS), or the treatment of conditions with drugs that result in immunodeficiency (e.g., cancer chemotherapeutic agents).

As used herein, "selecting" refers to the process of determining that an identified subject will receive an agent to prevent or treat the occurrence of a condition. Selecting can be based on an individuals susceptibility to a particular disease or condition due to, for example, family history, lifestyle, age, ethnicity, or other factors.

"Measuring" means detecting or determining the amount, for example, any one of an increase or a decrease in immunogenicity, an increase in an immune response, an increase in the concentration of dendritic cells at the site or irradiation, an increase in the maturation or activation of dendritic cells, an increase in the number of activated or mature dendritic cells, an increase in the production of HSPs, an increase in the secretion of HSPs, an increase in cell membrane permeability, an increase in the level of one or more cytokines or chemokines, an increase in antibody titer to an agent, or an increase in lymphocyte cytotoxic activity, according to the methods described herein. Measuring is the steps taken to detemine if an increase or decrease in a level of the material to be detected. Measuring may indicate a level that is zero or below the level of detection, or greater than the linear detection limit of the method used for measuring.

"Measuring" according to the disclosure is performed in vitro or in vivo, for example in the skin at the site of exposure to an electrical field, in serum or in blood or other biological sample, tissue or organ. "Measuring" also means detecting a change that is either an increase or decrease in the response (for example an increase in immunogenicity, an increase in an immune response, an increase in the concentration of dendritic cells at the site or irradiation, an increase in the maturation or activation of dendritic cells, an increase in the number of activated or mature dendritic cells, an increase in the production of HSPs, an increase in the secretion of HSPs, an increase in cell membrane permeability, an increase in the level of one or more cytokines or chemokines, an increase in antibody titer to an agent, or an increase in lymphocyte cytotoxic activity, according to the methods described herein, a decrease in the development of disease, or a decrease in the death rate in response to a pathogen challenge) of a subject to administration of an agent, according to the methods described herein.

"Measuring" is performed in a subject wherein an agent has been administered and wherein said subject has been exposed to an electrical field. "Measuring" is also performed in a control subject, for example a subject that has not received an agent and that has not been exposed to an electrical field, or a subject that has received an agent but has not been exposed to an electrical field.

As used herein, a "decrease" as it refers to a diminution in the level of a response as defined herein, means a response that is at least about 2-fold (for example about 2, 3, 4, 5, 10, 15, 20, 25, 30, 35, 40, 45, 50, 55, 60, 65, 70, 75, 80, 85, 90, 95, 100, 1000-fold or more) or at least about 2% (for example, about 2, 3, 4, 5, 10, 15, 20, 25, 30, 35, 40, 45, 50, 55, 60, 65, 70, 75, 80, 85, 90, 95, 99, or 100%), less than the level of response of an untreated subject, for example a subject that has not received an agent and has not been exposed to an electric field or a subject that has received an agent but has not been exposed to an electrical field.

The phrase "pharmaceutically acceptable carrier" is art recognized and includes a pharmaceutically acceptable material, composition or vehicle, suitable for administering compounds used in the methods described herein to subjects, e.g., mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. In the instant disclosure, suitable carriers to be administered prior to exposure to the electric field preferably do not include compounds that alter the absorption of heat by the tissue, or result in chemical reactions that can cause significant and/or irreversible damage to the cells or tissue. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

The language "therapeutically effective amount" or a "therapeutically effective dose" of a compound is the amount necessary to or sufficient to provide a detectable improvement in of at least one symptom associated or caused by the state, disorder or disease being treated. The therapeutically effective amount can be administered as a single dose or in multiple doses over time. Two or more compounds can be used together to provide a "therapeutically effective amount" to provide a detectable improvement wherein the same amount of either compound alone would be insufficient to provide a therapeutically effective amount.

As used herein, the terms "effective" and "effectiveness" includes both pharmacological effectiveness and physiological safety. Pharmacological effectiveness refers to the ability of the treatment to result in a desired biological effect in the patient. Physiological safety refers to the level of toxicity, or other adverse physiological effects at the cellular, organ and/or organism level (often referred to as side-effects) resulting from administration of the treatment. On the other hand, the term "ineffective" indicates that a treatment does not provide sufficient pharmacological effect to be therapeutically useful, even in the absence of deleterious effects, at least in the unstratified population. (Such a treatment may be ineffective in a subgroup that can be identified by the expression profile or profiles.) "Less effective" means that the treatment results in a therapeutically significant lower level of pharmacological effectiveness and/or a therapeutically greater level of adverse physiological effects, e.g., greater liver toxicity.

Thus, in connection with the administration of a drug, a drug which is "effective against" a disease or condition indicates that administration in a clinically appropriate manner results in a beneficial effect for at least a statistically significant fraction of patients, such as a improvement of symptoms, a cure, a reduction in disease load, reduction in tumor mass or cell numbers, extension of life, improvement in quality of life, or other effect generally recognized as positive by medical doctors familiar with treating the particular type of disease or condition.

In reference to response to a treatment, the term "tolerance" refers to the ability of a patient to accept a treatment, based, e.g., on deleterious effects and/or effects on lifestyle. Frequently, the term principally concerns the patients perceived magnitude of deleterious effects such as nausea, weakness, dizziness, and diarrhea, among others. Such experienced effects can, for example, be due to general or cell- specific toxicity, activity on non-target cells, cross-reactivity on non-target cellular constituents (non-mechanism based), and/or side effects of activity on the target cellular substituents (mechanism based), or the cause of toxicity may not be understood. In any of these circumstances one may identify an association between the undesirable effects and variances in specific genes from the subject to contact with an agent ex vivo.

Also in some instances, the method of selecting a treatment involves selecting a method of administration of a compound, combination of compounds, or pharmaceutical composition, for example, selecting a suitable dosage level and/or frequency of administration, and/or mode of administration of a compound. The method of administration can be selected to provide better, preferably maximum therapeutic benefit. In this context, "maximum" refers to an approximate local maximum based on the parameters being considered, not an absolute maximum.

Also in this context, a "suitable dosage level" refers to a dosage level that provides a therapeutically reasonable balance between pharmacological effectiveness and deleterious effects. Often this dosage level is related to the peak or average serum levels resulting from administration of a drug at the particular dosage level.

As used herein, a "cytokine" refers to a class of signaling proteins and glycoproteins that, like hormones and neurotransmitters, are used extensively in cellular communication. While hormones are secreted from specific organs to the blood, and neurotransmitters are related to neural activity, the cytokines are a more diverse class of compounds in terms of origin and purpose. They are produced by a wide variety of hematopoietic and non-hematopoietic cell types and can have effects on both nearby cells or throughout the organism, sometimes strongly dependent on the presence of other chemicals. The cytokine family consists mainly of smaller, water-soluble proteins and glycoproteins with a mass of between 8 and 30 kDa. Cytokines are critical to the functioning of both innate and adaptive immune responses. They are often secreted by immune cells which have encountered a pathogen as a way to activate and recruit more immune cells and increase the system's response to the pathogen. However, apart from their role in the development and functioning of the immune system, as well as their aberrant modes of secretion in a variety of immunological, inflammatory and infectious diseases, cytokines are also involved in several developmental processes during embryogenesis.

A cytokine useful according to the disclosure includes but is not limited to proinflammatory cytokines such as IL-1 alpha, IL-1 beta, TNF-alpha, IL-6, and IL- 12, as well as growth-promoting cytokines such as granulocyte-macrophage colony stimulating factor or type I and type II interferons.

As used herein, a "chemokine" refers to a family of small cytokines, or proteins secreted by cells. Proteins are classified as chemokines according to shared structural characteristics such as small size (they are all approximately 8-10 kilodaltons in size), and the presence of four cysteine residues in conserved locations that are key to forming their 3-dimensional shape. Their name is derived from their ability to induce directed chemotaxis in nearby responsive cells; they are chemotactic cytokines. Some chemokines are considered pro-inflammatory and can be induced during an immune response to promote cells of the immune system to a site of infection, while others are considered homeostatic and are involved in controlling the migration of cells during normal processes of tissue maintenance or development. Chemokines are found in all vertebrates, some viruses and some bacteria, but none have been described for other invertebrates. These proteins exert their biological effects by interacting with G protein-linked transmembrane receptors called chemokine receptors, are selectively found on the surfaces of their target cells!

A chemokine useful according to the disclosure includes but is not limited to IL- 8, MIP-I, MIP-2, IP-10, RANTES, CCR5, CCR6, CCR7, or CXCR4.

As used herein, "antibody titer" means a measurement of how much antibody an organism has produced that recognizes a particular epitope, expressed as the greatest dilution ratio (or its reciprocal) that still gives a positive result. In one instance, antibody titers are determined by ELISA assays.

As used herein, "lymphocyte cytotoxic activity" means the ability of a lymphocyte to kill another cell of a different type, typically by means of elaborating porins. This capability can be measured by co-culturing lymphocytes and target cells.

As used herein, "improving the efficiency" means increasing, as defined herein, any one of the level of response, the speed with which a response occurs, or the duration of the response, as compared to the response to an agent in a subject that has not been exposed to an electrical field. An increase in the efficiency refers to a change in the level of a response, the speed with which a response occurs or a duration of the response in a subject that has been exposed to an electric field as compared to a subject that has not been exposed to an electrical field, that is at least about 2-fold more (for example about 2, 3, 4, 5, 10, 15, 20, 25, 30, 35, 40, 45, 50, 55, 60, 65, 70, 75, 80, 85, 90, 95, 100, 1000-fold or more) or at least about 2% more (for example, about 2, 3, 4, 5, 10, 15, 20, 25, 30, 35, 40, 45, 50, 55, 60, 65, 70, 75, 80, 85, 90, 95, 99, or 100% more), than the efficiency of a response in a subject that has been exposed to an electrical field, as compared to an untreated subject.

As used herein, a subject that is "immunocompromised" refers to a subject that has an immunodeficiency of any kind. An "immunodeficiency" (or immune deficiency) is a state in which the immune system's ability to fight infectious disease is compromised or entirely absent. Most cases of immunodeficiency are acquired ("secondary") but some people are born with defects in the immune system, or primary immunodeficiency. An immunocompromised person may be particularly vulnerable to opportunistic infections, in addition to normal infections that could affect everyone. A subject that is "immunocompromised" refers to a subject wherein the decrease in the ability of the subject to respond to an infection or infectious disease is at least about 2-fold less (for example about 2, 3, 4, 5, 10, 15, 20, 25, 30, 35, 40, 45, 50, 55, 60, 65, 70, 75, 80, 85, 90, 95, 100, 1000-fold or more) or at least about 2% less (for example, about 2, 3, 4, 5, 10, 15, 20, 25, 30, 35, 40, 45, 50, 55, 60, 65, 70, 75, 80, 85, 90, 95, 99, or 100%), than the ability of a subject that is not immunocompromised to respond to an infection or an infectious disease.

As used herein, "extracellular matrix" or "ECM" refers to the extracellular part of tissue that usually provides structural support to the cells in addition to performing various other important functions. The extracellular matrix is the defining feature of connective tissue in mammals. Extracellular matrix includes the interstitial matrix and the basement membrane. Interstitial matrix is present between various cells (i.e., in the intercellular spaces). Gels of polysaccharides and fibrous proteins fill the interstitial space and act as a compression buffer against the stress placed on the ECM. Basement membranes are sheet-like depositions of ECM on which various epithelial cells rest. The extracellular matrix comprises polysaccharides (for example, glycosaminoglycans or cellulose) and proteins (such as collagen) secreted by cells. Examples of extracellular matrix proteins include but are not limited to collagen, laminin, fibronectin, vitronectin, elastin, glycosaminoglycans, proteoglycans, and combinations of some or all of these components.

As used herein, "skin cells" are cells which make up the epidermis and dermis for example, Merkel cells, keratinocytes, melanocytes and dendritic cells.

As used herein, "secretion" refers to the process of segregating, elaborating, and releasing a substance from a cell.

As used herein, "obtaining" is understood as purchase, procure, manufacture, or otherwise come into possession of the desired material.

"Providing," refers to obtaining, by for example, buying or making the, e.g., polypeptide, drug, polynucleotide, probe, antigen, and the like, including libraries of such compounds or libraries of combinations of types of compounds. The material provided may be made by any known or later developed biochemical or other technique. For example, compounds may be derived from natural sources, be chemically synthesized by directed or combinatorial methods, or a collection of known compounds (e.g., compounds approved for therapeutic use in humans).

As used herein, the term "recombinant DNA molecule" as used herein refers to a DNA molecule, which is comprised of segments of DNA joined together by means of molecular biological techniques.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

Unless specifically stated or obvious from context, as used herein, the term "or" is understood to be inclusive.

Unless specifically stated or obvious from context, as used herein, the terms "a", "an", and "the" are understood to be singular or plural.

As used herein, "about" is understood to be relative to the amount of variance typically tolerated in the specific assay, method, or measurement provided. For example, "about" is typically understood to be within about 3 standard deviations of the mean, or two standard deviations of the mean. About can be understood as a variation of 20%, 15%, 12%, 10%, 8%, 5%, 3%, 2%, or 1%, depending upon the tolerances in the particular art, device, assay, or method.

The recitation of a listing of chemical groups in any definition of a variable herein includes definitions of that variable as any single group or combination of listed groups. The recitation of an embodiment or instance for a variable or aspect herein includes that embodiment or instance as any single embodiment or instance or in combination with any other embodiments or instances or portions thereof.

Any compositions or methods provided herein can be combined with one or more of any of the other compositions and methods provided herein.

### DETAILED DESCRIPTION OF THE INVENTION

The disclosure describes a method of exposing the skin or other mucosa to an ultrashort, high energy, pulsed electrical fields (usHEPEFs) to enhance the immunostimulatory effects of vaccines without causing visible skin or mucosal damage and without causing significant and irreversible damage to either tissues or cells. Without wishing to be bound by mechanism, it is suggested that these electrical fields stimulate the extracellular release of HSP70 from cells in the cells of the epithelial layers, alter the intracellular signaling activity of dendritic cells and increase the concentration and activity of immune cells in the area at or near exposure to the electrical field. The vaccine-enhancing effect requires a device suitable of delivering a sufficient number of discharges of a defined field strength and pulse duration to the skin or mucosa of the subject necessary over a relatively short exposure time without causing concomitant tissue damage necessary to induce extracellular release of HSP70 in the epidermal and dermal layer of the skin or in the epithelial layers of mucosal linings of the body and induce a detectably higher level of immunogenicity to an administered vaccine or antigen, or a detectably higher immune response to vaccination.

The disclosure describes methods for enhancement of vaccine efficacy by exposing the skin or mucosa to electrical fields. Fundamentally, it utilizes a PEVA device sufficient to provide the necessary electric field strength and pulse duration. It is suggested that exposure of the skin or mucosal tissues to a sufficient series of discharges from an electric field of sufficient voltage and pulse durations under the specific parameters disclosed herein causes enough stress on cells of the exposed epithelial layers sufficient to result in the secretion of a significant amount heat shock protein 70 molecules into the extracellular matrix, the alteration of internal cell signaling in dendritic cells and concentration and activation of dendritic cells in the area of the electric field exposure, but not enough to result in irreversible cellular damage to the vast majority of skin cells. The end result of this electrical exposure is the enhanced response of the skin immune mechanisms to administered vaccines, leading to a better immune response in the immunized subject.

The parameters of exposure of cells to usHEPEFs sufficient to induce intracellular effects but below the level that causes irreversible plasma or organelle membrane rupture and subsequent cell death have been established by practioners of the art using a variety of cellular models. Beebe et al. (2004) found that the thresholds for breaching plasma membrane integrity in HL-60 and Jurkat cells were between 26 and 40 kV cm-1 for 60 nanosecond pulses but rose to between 150 and 300 kV/cm for 10 nanosecond pulses. In normal cells, the very short duration of the electrical pulses make it very difficult to irreversibly porate the membranes so as to cause cell destruction. However, multiple electrical discharges could lead to accumulation of pores that would be fatal (U.S. Patent 6,326,177 B1). In the case of abnormal cells, such as cancer cell lines, pulse discharges similar to those described to cause enhancement of immune response (between 20-300 kV/cm for pulse durations between 10-300 nanoseconds) can result in apoptosis. Practioners of the art have utilized such electric fields to cause apoptosis or necrosis of cells (U.S. Patent 6,326,177 B1; U.S. Patent Application 2002/0010491 A1).

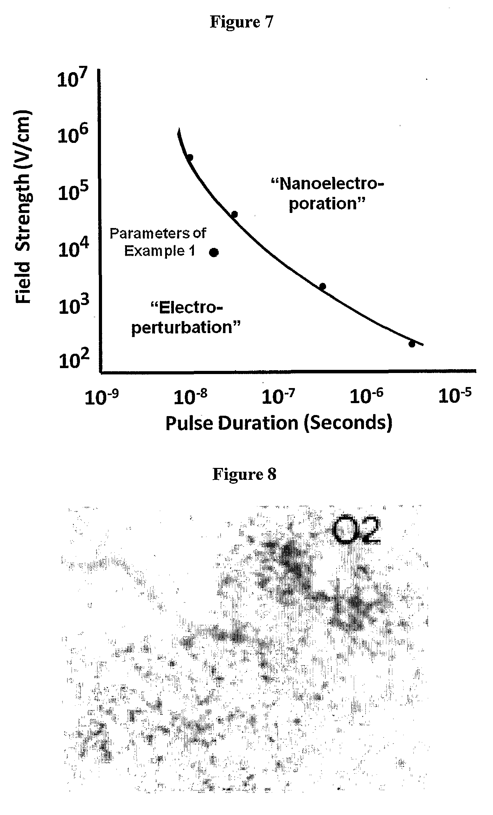

As a result of these efforts, it is generally accepted by practitioners that in the nanosecond (10⁻⁷ to 10⁻⁹ second) range of pulse durations, minimum electrical voltage intensities on the order of 10³ to 10⁷ volts per centimeter are required to generate intracellular effects. For nanoelectroporation, the required voltage intensities are inversely proportional to the duration of the pulse. Within these broad parameters, electrical fields of intensities of 10-350 kV/cm and pulse durations of hundreds of picoseconds to 60 nanoseconds have been utilized to alter intracellular signaling without causing apoptosis or necrosis of the cell (U.S. Patent Application 2003/0170898 A1; U.S. Patent Application 2006/0161221 A1). Figure 7 illustrates the inverse relationship between electric field strength and pulse duration required for nanoelectroporation based on a series of mathematical modeling studies and empirical experiments from the literature, and it illustrates the domain of effects related primarily to nanoelectroporation and to electroperturbation. In contrast to examples related to nanoelectroporation effects, the exposures used to generate immune enhancing effects are below that required for nanoelectroporation. In general, the ideal ranges of field strength and pulse duration will be below the threshold to cause nanoelectroporation.

To practice the methods of the disclosure, the skin or mucosa must be exposed to the electric field at a site at or near the site of vaccine or antigen administration. Preferably, the site of administration is exposed to the electrical field. The skin or mucosa can be exposed to the electric field at a site near the administration site provided that the skin or mucosa exposed to the electric field is within the area in which dendritic cells affected by the electric field or its aftermath would be active.

The non-damaging method of exposure of tissue or mucosa to the electric field of the disclosure is further distinct from known applications of high-energy pulsed electrical fields in that it does not cause apoptosis or necrosis to the vast majority of cells exposed to the electric field. The methods provided herein are not based on creating irreversible damage or destruction of cells such as those described in other examples (US Patent 6,326, 177 and US Patent Application 2002/0010491). Instead the methods are based on exposing cells to an electric field of sufficient voltage and pulse frequency to cause secretion of heat shock protein, to alter intracellular signaling of immune cells, and to concentrate activated immune cells in the area of the electric field discharge without causing significant or irreversible damage to the cells. Irreversible damage does not include a cell stress response in which the cell may enter a period of senescence prior to cell division. Reversible damage can be induced and detected, for example, by the activation of one or more cell cycle checkpoints which can result in a pause in the cell cycle or by the detection of a transient increase in membrane permeability.

Irreversible damage to cells can be caused by triggering the release of destructive molecules within the cell such as radical oxygen or nitrogen species, by triggering signaling cascades that induce apoptosis in healthy cells, such as through the caspase-activation pathways, by causing significant damage to the DNA of the cell, or by causing irreversible poration in the membranes of cells or their organelles that lead to the death of the cell.

Irreversible damage to the cells can result from the release of an excess amount of destructive molecules within a cell. It is known that electroporation through pulsed electric fields can lead to the generation of radical oxygen products. While the release of ions within the cell such as Ca2+ in response to usHEPEFs have been described, it is not known that these generate sufficient reactive molecules to trigger cell death.

Irreversible damage to the cells can result from the induction of cell signaling processes that lead to apoptosis. While lower frequency pulsed electrical pulse fields of sufficient field strength act almost exclusively on cell membranes and can result in poration of cell membranes, usHEPEFs of sufficient field strength can allow passage of ions through intracellular membranes that can trigger apoptotic signaling cascades, often through caspase-based signaling and release of cytochrome c. While these cascades are normally mitigated by other components within the cell such as heat shock proteins, in some cells, particularly those that are abnormal such as cancer cells, these signaling cascades can result in apoptosis.

An alternative method of causing irreversible tissue destruction is through the irreversible poration of the membrane of a cell or its organelles. Under appropriate experimental conditions, application of high-energy pulsed electrical fields to cell membranes will result in the development of pores that result in membrane permeability. Below a defined threshold these pores are formed reversibly, disappearing in minutes or seconds. However, when the field strength of or duration of the electric field applied to the membrane induces the formation of pores that are not reversible, the result is the loss of cytoplasm and the death of the cell. With pulse durations in the nanosecond range and below, single electrical pulses are unlikely to cause irreversible damage, whereas a number of such pulses could have an additive effect, leading to irreversible intracellular poration.

Yet another method of causing irreversible tissue destruction is through application of sufficient number of ultrashort electric field pulses sufficient to induce changes in intracellular signaling, alteration of metabolic pathways, or activation or deactivation of genes that result in the apoptosis of the cell (U.S. Patent Application 2003/0170898; U.S. Patent Application 2006/0062074). In these cases the number of pulse repetitions required to induce apoptosis were typically in the range of 20-50. In addition, the cell lines investigated comprised either metabolically abnormal cells, such as tumor cells, or highly specialized cells, such as astrocytes. It is likely that the response of normal dermal or mucosal cells would be quite different from these cell lines.

In contrast to examples cited above, exposures of many seconds typically involving 10,000 or more distinct nanosecond pulses are used to enhance immune response to vaccine, but without irreversible tissue damage.

Still another method of causing irreversible tissue destruction is the direct damage of cellular DNA by electrical pulses. Investigators have reported empirical data that shows that some abnormal cell types, such as cancer cells, exposed to usHEPEFs may incur DNA damage.

Irreversible damage to cells from the effects of high-energy pulsed electrical fields result in either the apoptosis or necrosis of the cell, depending on the site and extent of the damage, or the disruption of the entire tissue (in most cases from excessive heating of tissues by the electrical energy). Cells need not be killed immediately upon exposure to the electrical field, but instead, they may die over a longer period of time as a direct result of exposure to an electric field (e.g., initiate apoptosis, become necrotic, somatic fusion and subsequent mitotic catastrophe). The exact cause or causes of irreversible damage is not a limitation of the disclosure. Irreversible damage can result from a combination of mechanisms.

Measurement of the concentration of viable cells in a particular tissue or culture is routine in the art. Methods and kits are known in the art to detect apoptosis and necrosis. The number of cells undergoing apoptosis or necrosis can be readily scored and expressed as a percentage of cells exposed to the electrical field.

Apoptosis can be detected using flow cytometry techniques. For example, apoptotic cells show an increased uptake of the vital dye HO342 compared to live cells due to a changes in membrane permeability. Apoptosis can be measured using a number of other assay-based approaches including measurement of DNA fragmentation, membrane phospholipid changes, interleukin-1 beta converting enzyme-like protease activation, or nucleosomal fragmentation by DNA agarose gel electrophoresis. Finally, apoptosis can be detected through visual means such as changes in cell morphology.

Necrotic cells can be detected by flow cytometry techniques, such as the addition of the nucleic acid stain PI, which binds to DNA or RNA but cannot permeate cell membranes and therefore is visible under fluorescence only if the cell membrane has been compromised, or stains such as propidium iodide or 7-AAD that discriminate cells which have lost membrane integrity. In addition, they can be visualized optically using standard staining and microscopy techniques.

The method of this disclosure utilizes exposure of tissue to high energy pulsed electrical fields, but is designed to avoid significant and irreversible damage to cells. Preferably less than 1% of cells are irreversibly damaged upon exposure to an electric field using the methods of the disclosure.

Finally, the method is distinguished from the use of relatively low power electric field in the kilovolt per meter range of field strength that have been shown to induce a variety of therapeutic effects such as relief of pain, accelerate bone and wound healing, decrease inflammation and enhance blood circulation. These approaches are hypothesized to induce a resonant coupling between the electric field and the cell membrane, leading to signal transduction processes.

Without being bound by mechanism, the approach described below demonstrates that a brief exposure of the skin with a PEVA device that delivers a series of ultrashort pulses of a high powered electric field with the proper combination of working characteristics leads to enhanced and rapid secretion of HSP70 by skin cells, alteration of the activity of dendritic cells, and concentration of active dendritic cells at the site of electric field exposure, and results in a significant improvement in response to vaccination without causing significant irreversible damage to the irradiated cells.

### Immunostimulation through Heat Shock Synthesis and Release

Subjecting tissues to physiologic levels of stress results in the mobilization and overproduction (or overexpression) of heat shock proteins (HSPs) by skin cells (heat shock response). In general, cells produce HSPs in response to stress caused by heat, poisons or signals from nerves or hormones. There are five major groups of HSPs found in humans based on molecular size: 20-30, 50-60, 70, 90, and 100-110 kDa. Within the cell, heat shock proteins play an important protective role against these stresses, specifically assisting in protein maintenance, folding, chaperoning and degredation. and, when expressed on the surface of the cell, assist with the stabilization of the cell membrane.

Extracellularly, heat shock proteins, in particular the 70 kDa HSP70, have several positive effects on immune function. While HSP70 is involved in protein folding and chaperoning activities inside the cell, outside the cell it acts as a chaperone of protein antigens to dendritic cells and stimulates their maturation. Dendritic cells are key actors in the immune response against both infectious pathogens and cancerous tumors. In an immature state, dendritic cells capture and process antigens with high efficiency. After maturation, they migrate to lymphoid organs to present these antigens, as well as co-stimulatory molecules, to T cells. Approximately 40% of the body's antigen-presenting dendritic cells are located in the skin.

While it is known that heat results in the increased expression of HSP70 within the cell, the application of heat can also lead to secretion of greater quantities of HSP70 outside of viable cells. Just as cells vary in their ability to express HSP70, they also differ in their ability to secrete HSP70. Some cancer cells and some specialized cells such as glial cells are able to secrete HSP70 at a much higher quantities. A number of hypotheses for the mechanism of secretion have been proposed, including transport by lipid rafts, small vesicles, secretory granules or through an endolysosomal compartment. Research conducted by Evdonin et al. (2006) showed that heating of skin keratinocyte cells resulted in secretion of HSP70 through vesicular transport at the initial stages of heating. Other techniques have been demonstrated in the art to induce HSP70 secretion from cells without causing the apoptosis or necrosis of the cells, including application of phospolipase c inhibitors, laser exposure (Russian Patent Application 2007113393), and transfection of HSP70 DNA for overexpression in monocytes.

This method utilizes a high-energy, ultrashort pulsed electric field to rapidly and non-destructively (i.e., without significant or irreversible damage) cause the secretion of significant additional quantities of HSP70 from the cells of the skin or mucosa (Figure 8). This effect of HSP secretion is not related to tissue heating, since it has already been established that the temperature rise required to achieve intended effects in cells and tissues with nsHEPEFs is in the range of 2-4° C. Mild heating of tissues and cells at that level in general does not lead to overexpression or secretion of HSPs in the heated cells.

### Activation of Dendritic Cells

The secretion of HSP70 introduces a significant additional number of chaperone proteins into the surrounding epidermal tissues that are capable of enhancing presentation the protein antigens from a vaccine to Langerhans cells. HSP70 is capable of inducing maturation of immature dendritic cells, rapidly multiplying the number that migrates to the lymph system, where they prime T-cells to mount an immune response against a cell or virus expressing the vaccine antigen. Immature dendritic cells thus stimulated are better able to process and express vaccine antigen and produce chemokine or cytokine signals to other immune cells that can enhance vaccine response. Dendritic cells that have already been activated are assisted in their process of maturation and migration.

Ultrashort, high energy pulsed electric fields may also induce calcium ion fluxes within dendritic cells at the site of electrical discharge that trigger enhanced immune activity. It has been described that usHEPEFs may result in the flux of calcium ions from within cellular organelles such as the endoplasmic reticulum. These fluxes have been described to have a variety of effects including induction of platelet aggregation, modulation of neurotransmitter release from nerves, increasing muscle contraction, stimulating hormone release, stimulating cell respiration and proliferation, and improving the transcription of genes (US Patent Application 2006/0161221). Calcium plays a role in intracellular signaling of dendritic cells. Increases in cytosolic Ca²⁺ result in the increased binding of Ca²⁺ to calmodulin kinase II, stimulating calmodulin kinase II activity and leading to the activating of the MHC-II pathway in dendritic cells. Additionally, calcium fluxes can stimulate the dendritic cell toward maturation and antigen presentation.

Additionally, the present invention leads to the concentration of dendritic cells in the local area of the skin or mucosa exposed to an electrical field. This concentration also serves to increase the effectiveness of vaccination by increasing the number of dendritic cells available to receive vaccine antigens and migrate to the lymph system after maturation.

It is believed that the concentration of dendritic cells in the area of exposed to the electric field brings them into proximity with the elevated levels of secreted HSP70 proteins, leading to an acceleration and augmentation of the processes of antigen uptake, dendritic cell maturation, and migration of activated dendritic cells toward the lymphatic system. In addition, usHEPEFs may directly stimulate dendritic cells to be more responsive to antigen presentation, especially when chaperoned by heat shock protein. This production of heat shock proteins and concentration of dendritic cells in the same area in the skin is suggested to make electrical stimulation of the skin a powerful adjuvant approach that is not burdened by the kind of safety issues that plague current vaccine adjuvants.

### EXAMPLES

### Example 1: Effects of usHEPEFs on the skin in vivo

Healthy white CBA mice were treated using a GVIS-CH semiconductor-based electric pulse generator with coaxial electrodes. The generator produced 30 nanosecond pulses at 9 Kv/cm at a frequency of 250 Hertz. Using the electrodes with a gap of approximately 5 mm, the electric field was applied to the skin of the ventral side of one ear on each mouse. This area was exposed to the pulsed electric field for 60 seconds. Five minutes after exposure to the electric field, 50 microliters of Vaxigrip (Sanofi Pasteur) influenza vaccine was injected into the ventral ear skin in the area of the electric field exposure. The remaining ear of each mouse acted as a control for the experiment.

One hour after exposure to electric field and vaccination, the mice were sacrificed. Immunohistochemical staining using antibodies to HSP70 (Russian Patent 2242764) on the ventricular epidermal sheets of the ear provided a qualitative estimate of the increased levels of the secreted HSP70 as compared to the tissue from untreated ear from the same animal. Staining showed that the electric field exposure increased the secretion of HSP70 into the extracellular space by 3.1 times compared with the control ears (Figure 8). This effect was found to be dose dependent. Exposures of 60 and 90 seconds produced a greater secretion of HSP70 than exposures of 30 seconds.

Exposure of the skin to the electric field was safe and did not produce any visible or microscopic signs of burn injury or tissue de-epithelization, but resulted only in very mild intermittent hyperemia confined to the exposure area.

### Example 2: Enhanced antibody production in response to a vaccine administered with electric field exposure

Healthy white CBA mice were treated using a GVIS-CH semiconductor-based electric pulse generator with coaxial electrodes. The generator produced 30 nanosecond pulses at 9 Kv/cm at a frequency of 250 Hertz. Using the electrodes with a gap of approximately 5 mm, the electric field was applied to the skin of the ventral side of one ear on each mouse. This area was exposed to the pulsed electric field for 60 seconds. Five minutes after exposure to the electric field, 50 microliters of Vaxigrip (Sanofi Pasteur) influenza vaccine was injected into the ventral ear skin in the area of the electric field exposure. A separate set of mice received 50 microliters of Vaxigrip vaccine only in the ventral skin of the ear. A third group of mice served as normal controls. Bloods were drawn from each group before the study and 28 days after vaccination. Mice receiving the electric field exposure along with vaccination had antibody titers 2.26 times higher than mice receiving only vaccine.

Exposure of the skin to the electric field was safe and did not produce any visible or microscopic signs of burn injury or tissue de-epithelization, but resulted only in very mild intermittent hyperemia confined to the exposure area.

### Example 3: Stimulation of response to flu vaccine in immunocompromised humans with electric field pulse

In a three-arm study, two groups of vaccine non-responding patients subcutaneously receive either a standard dose of an approved seasonal influenza vaccination (Vaxigrip or other) or the same method of vaccination after a one-minute exposure of the skin to an ultrashort, pulsed electric field vaccine adjuvant (PEVA). Nonresponders for the study are identified by a lack of clinically-accepted measurements of vaccine response, including antibody titers 30-60 days after immunization below the threshold that is considered protective, such as a hemagglutinin inhibition antibody geometric mean titers (GMTs) of greater than 1:40. An electric field pulse generator is used that produces 30 nanosecond pulses at 9 Kv/cm at a frequency of 250 Hertz. PEVA is applied to the skin of the arm using a set of electrodes with a gap of approximately 5 mm. This area is exposed to the pulsed electric field for 60 seconds.

A third group of healthy control subjects receive standard vaccination. Three weeks after vaccination, antibody ELISA tests are performed on all subjects. Approximately 80% of healthy subjects show significant response to vaccination based on these measurements (e.g., hemagglutinin inhibition antibody GMTs greater than 1:40), as opposed to 20% of vaccine responders only receiving vaccination. More than 50% of nonresponders receiving vaccine after exposure electric field show a response to vaccination, with antibody titers close to that of normal controls.

Exposure of the skin to the electric field was safe and did not produce any visible or microscopic signs of burn injury or tissue de-epithelization, but resulted only in very mild intermittent hyperemia confined to the exposure area.

### Example 4: Protection of the elderly in annual influenza vaccination

In this study on the effect of PEVA on protection of older individuals from seasonal influenza, healthy adults ages 65 and over are randomized into three different study arms. Arm 1 subjects receive the standard of care-intramuscular vaccination with the 45 micrograms of influenza vaccine antigen manufactured to provide prophylaxis against the current season's influenza strain. Arm 2 subjects receive with the same vaccine in an intradermal delivery (e.g., 36 micrograms of vaccine antigen delivered with the Soluvia intradermal microinjection system, Becton, Dickinson & Company). Arm 3 subjects receive the same vaccine in an intradermal dosage coupled with a PEVA immediately before administration of the vaccine. An electric field pulse generator is used that produces 30 nanosecond pulses at 9 Kv/cm at a frequency of 250 Hertz. PEVA is applied to the skin of the arm using a set of electrodes with a gap of approximately 5 mm. This area is exposed to the pulsed electric field for 60 seconds. Sufficient numbers of patients are enrolled in each arm to ensure sufficient statistical powering at the end of the study, and enrollees in each arm are case-matched demographically. Enrollment is conducted at the start of the influenza season (November to May).

After 30 days, blood samples are drawn from each patient and assessed for influenza antibody titers. Patients are followed over the influenza season (approximately 6 months) and statistics are recorded for clinical activity related to influenza (clinical visits, emergency room visits, hospitalizations, etc.).

The study shows that combination of PEVA and intradermal vaccination is statistically superior to both standard of therapy vaccination and epidermal vaccination alone in the improvement of antibody titers, and that patients receiving both the epidermal vaccination and PEVA treatment have a statistically significant decrease in clinical events related to influenza, particularly with respect to hospitalization.

### Example 5: Dose-sparing in avian influenza vaccination

In this study of the dose-sparing effects of PEVA when combined with intradermal vaccination against the avian influenza vaccine (H5N1 vaccine, Sanofi Aventis), groups of healthy mice are randomized into four major groups. Mice in group 1 receive the standard intramuscular avian influenza vaccine. Mice in group 2 receive the standard avian influenza vaccine administered at 20% of the intramuscular dose intradermally in the ear. Mice in group 3 receive epidermal avian influenza vaccine at 20% of the intramuscular dose after a one minute PEVA treatment. Parameters for PEVA are the same as those in other examples.

After 30 days, blood samples are drawn from each mouse and assessed for H5N1 antibody titers. The study shows that intradermal vaccination with PEVA is statistically superior to both intradermal vaccination without PEVA as well as intramuscular vaccination.

### Example 6: Potentiation of a DNA Vaccine

Four sets of healthy mice are assessed for the difference in response to a novel DNA-based Hepatitis B vaccine compared with an existing, approved hepatitis B vaccine (Recombivax HB®, Merck and Co.). Mice in the first group receive the Recombivax HB vaccine at a dose tittered to provide serologic correlates of protection. Mice in the second and third groups both receive an intradermal vaccination with the DNA vaccine. Mice in the third group receive a one minute PEVA treatment 24 hours after the initial injection of the vaccine. A fourth group of mice act as controls.

Blood is drawn from all mice at baseline and assessed for antibody titers against the hepatitis B surface antigen, and again four weeks after the initial vaccination. The study shows that mice receiving the PEVA treatment had a statistically significant increase in protective antibodies compared to DNA vaccination alone, and that these levels are at least statistically comparable to immunization with the Recombivax HB vaccine.

### Example 13: Potentiation of a Recombinant Subunit Vaccine

A population of healthy individuals is assessed for the difference in response to a novel subunit-based MMR (measles, mumps, rubella) vaccine compared with an existing, approved MMR vaccine that is composed of attenuated live viruses (MMR II®, Merck and Co.). Subjects are randomized to receive intredermal vaccination either with the subunit-based MMR vaccine, the subunit MMR vaccine combined with PEVA, or the standard MMR vaccine. Administration of each type of vaccine, including dosage, frequency of vaccination, and methods of administration, follows FDA-approved approaches for intradermal vaccination with the MMR. Sufficient numbers of patients are enrolled in each arm to ensure sufficient statistical powering at the end of the study, and enrollees in each arm are case-matched demographically. Exclusion criteria for the study includes previous vaccination against or exposure to measles, mumps, or rubella. The time of exposure to PEVA is about one minute.

After completion of the vaccination period, blood samples are drawn from each patient and assessed for seroconversion against measles, mumps and rubella.

The study shows that combination of PEVA and subunit vaccination yields statistically significant improvement in antibody titers over subunit vaccination alone and is statistically equivalent or superior to FDA-approved subunit vaccination.

### Example 12: Potentiation of a Synthetic Peptide Vaccine

A population of healthy individuals is assessed for the difference in response to a novel synthetic peptide vaccine against bacterial meningitis compared with an existing, approved bacterial meningitis vaccine that is composed of meningococcal polysaccharides conjugated to the diphtheria toxoid (MENACTRA®, Sanofi Pasteur). Subjects are randomized to receive either the synthetic peptide vaccine, the synthetic peptide vaccine combined with PEVA, or the standard conjugated vaccine. Administration of each type of vaccine, including dosage, frequency of vaccination, and methods of administration, follows FDA-approved approaches. The time of exposure to PEVA is about one minute. Sufficient numbers of patients are enrolled in each arm to ensure sufficient statistical powering at the end of the study, and enrollees in each arm are case-matched demographically. Exclusion criteria for the study includes previous vaccination against or exposure to bacterial meningitis.

After completion of the vaccination period, blood samples are drawn from each patient and assessed for seroconversion against bacterial meningitis.

The study shows that combination of PEVA and synthetic peptide vaccination yields statistically significant improvement in antibody titers over peptide vaccination alone and is statistically equivalent or superior to FDA-approved conjugated vaccination.

## Claims

1. Use of an antigen in the manufacture of a vaccine, wherein the vaccine is for administration in combination with a pulsed electrical field of sufficient voltage and pulse frequency for enhancing an immunostimulatory effect of the vaccine as compared to the vaccine used without a pulsed electrical field, but below a threshold of electroporation of cell membranes, and wherein the administration comprises exposing an area of skin or mucosa of a subject to ultrashort, high energy, pulsed electrical fields, and contacting the subject with the antigen in the layers of the skin or mucosa at or near the site of the electrical field.

2. The use according to Claim 1, wherein the electrical field does not cause apoptosis or necrosis to more than 1 % of the cells.

3. The use according to Claim 1, wherein the duration of the electrical field pulses is under 100 nanoseconds.

4. The use according to Claim 1, wherein the electrical field ranges from about 1 kV/cm to about 300 kV/cm.

5. An antigen for use in a vaccine, wherein the vaccine is for administration in combination with a pulsed electrical field of sufficient voltage and pulse frequency for enhancing an immunostimulatory effect of the vaccine as compared to the vaccine used without a pulsed electrical field, but below a threshold of electroporation of cell membranes, and wherein the administration comprises exposing an area of skin or mucosa of a subject to ultrashort, high energy, pulsed electrical fields, and contacting the subject with the antigen in the layers of the skin or mucosa at or near the site of the electrical field.

6. An antigen for use in a vaccine according to Claim 5, wherein the duration of the electrical field pulses is under 100 nanoseconds.

7. An antigen for use in a vaccine according to Claim 5, wherein the electrical field ranges from about 1 kV/cm to about 300 kV/cm.

## Patentansprüche

1. Verwendung eines Antigens bei der Herstellung eines Impfstoffs, wobei der Impfstoff zur Verabreichung in Verbindung mit einem gepulsten elektrischen Feld von ausreichender Spannung und Pulsfrequenz dient, um eine immunstimulierende Wirkung des Impfstoffs im Vergleich zu dem ohne ein gepulstes elektrisches Feld verwendeten Impfstoff zu verbessern, aber unterhalb einer Schwelle von Elektroporation von Zellmembranen, und wobei die Verabreichung das Aussetzen eines Haut- oder Schleimhautbereichs eines Subjekts an ultrakurze, gepulste, elektrische Hochenergiefelder und das Inkontaktbringen des Subjekts mit dem Antigen in den Lagen der Haut oder Schleimhaut an oder nahe der Stelle des elektrischen Feldes umfasst.

2. Verwendung nach Anspruch 1, wobei das elektrische Feld keine Apoptose oder Nekrose an mehr als 1 % der Zellen verursacht.

3. Verwendung nach Anspruch 1, wobei die Dauer der Pulse des elektrischen Feldes unter 100 Nanosekunden liegt.

4. Verwendung nach Anspruch 1, wobei das elektrische Feld von etwa 1 kV/cm bis etwa 300 kV/cm reicht.

5. Antigen zur Verwendung in einem Impfstoff, wobei der Impfstoff zur Verabreichung in Verbindung mit einem gepulsten elektrischen Feld von ausreichender Spannung und Pulsfrequenz dient, um eine immunstimulierende Wirkung des Impfstoffs im Vergleich zu dem ohne ein gepulstes elektrisches Feld verwendeten Impfstoff zu verbessern, aber unterhalb einer Schwelle von Elektroporation von Zellmembranen, und wobei die Verabreichung das Aussetzen eines Haut- oder Schleimhautbereichs eines Subjekts an ultrakurze, gepulste, elektrische Hochenergiefelder und das Inkontaktbringen des Subjekts mit dem Antigen in den Lagen der Haut oder Schleimhaut an oder nahe der Stelle des elektrischen Feldes umfasst.

6. Antigen zur Verwendung in einem Impfstoff nach Anspruch 5, wobei die Dauer der Pulse des elektrischen Feldes unter 100 Nanosekunden liegt.

7. Antigen zur Verwendung in einem Impfstoff nach Anspruch 5, wobei das elektrische Feld von etwa 1 kV/cm bis etwa 300 kV/cm reicht.

## Revendications

1. Utilisation d'un antigène dans la fabrication d'un vaccin, le vaccin étant destiné à l'administration en association avec un champ électrique pulsé de tension et fréquence d'impulsion suffisantes pour augmenter un effet immunostimulant du vaccin par rapport au vaccin utilisé sans champ électrique pulsé, mais en-dessous d'un seuil d'électroporation des membranes cellulaires, et l'administration comprenant l'exposition d'une surface de peau ou de muqueuse d'un sujet à des champs électriques pulsés, ultra-courts, à haute énergie, et la mise en contact du sujet avec l'antigène dans les couches de la peau ou de la muqueuse sur le site du champ électrique ou à proximité de ce dernier.

2. Utilisation selon la revendication 1, dans laquelle le champ électrique ne provoque pas l'apoptose ou la nécrose de plus de 1 % des cellules.

3. Utilisation selon la revendication 1, dans laquelle la durée des impulsions de champ électrique est inférieure à 100 nanosecondes.

4. Utilisation selon la revendication 1, dans laquelle le champ électrique est compris entre environ 1 kV/cm et environ 300 kV/cm.

5. Antigène pour l'utilisation dans un vaccin, le vaccin étant destiné à l'administration en association avec un champ électrique pulsé de tension et fréquence d'impulsion suffisantes pour augmenter un effet immunostimulant du vaccin par rapport au vaccin utilisé sans champ électrique pulsé, mais en-dessous d'un seuil d'électroporation des membranes cellulaires, et l'administration comprenant l'exposition d'une surface de peau ou de muqueuse d'un sujet à des champs électriques pulsés, ultra-courts, à haute énergie, et la mise en contact du sujet avec l'antigène dans les couches de la peau ou de la muqueuse sur le site du champ électrique ou à proximité de ce dernier.

6. Antigène pour l'utilisation dans un vaccin selon la revendication 5, la durée des impulsions de champ électrique étant inférieure à 100 nanosecondes.

7. Antigène pour l'utilisation dans un vaccin selon la revendication 5, le champ électrique étant compris entre environ 1 kV/cm et environ 300 kV/cm.
